# EUROPEAN PATENT APPLICATION

(11) **EP 2 127 647 A1**
(43) Date of publication of application: **02.12.2009**
(21) Application number: 07706809.6
(22) Date of filing: 16.01.2007
(51) Int. Cl.: A61K 31/133, A61K 31/164, A61K 35/74, A61P 21/00

(54) **COMPOSITION FOR INHIBITING MUSCLE DAMAGE**

(71) Applicant: Mizkan Group Corporation, Handa-shi, Aichi 475-8585 (JP)
(72) Inventor: SAKAKIBARA, Rena, Handa-shi Aichi 475-0862 (JP); SUGIYAMA, Kenichi, Handa-shi Aichi 475-0928 (JP)
(74) Representative: Gille Hrabal Struck Neidlein Prop Roos
(86) International application number: PCT/JP2007/050482
(87) International publication number: WO 2008/087705

(57) **Abstract**

The present invention aims to provide a composition for protecting a muscle damage having high safety and a stronger muscle damage-protecting effect. The present invention provides a composition for protecting a muscle damage comprising, as an active ingredient, an extract with a lipid-soluble organic solvent of an acetic acid bacterium or preferably an extract with ethyl acetate of an acetic acid bacterium, alkali-stable lipid of an acetic acid bacterium, N-acylsphinganine, sphinganine, amino lipids, or terpenoid compounds. Further, the present invention provides a composition for protecting a muscle damage comprising, as an active ingredient, an extract with a lipid-soluble organic solvent of a vinegar or preferably an extract with ethyl acetate of a vinegar.

## Description

### Technical Field

The present invention relates to a composition for protecting a muscle damage caused by excise and the like, and more specifically, to a composition for protecting a muscle damage comprising, as an active ingredient, an extract with a lipid-soluble organic solvent of an acetic acid bacterium or a vinegar. Further, the present invention also relates to a composition for protecting a muscle damage comprising, as an active ingredient, an alkali-stable lipid of an acetic acid bacterium, N-acylsphinganine, sphinganine, amino lipids, or terpenoid compounds in the alkali-stable lipid.

### Background Art

All physical activities in daily life such as walking for domestic work, gardening, or commuting, and hobby and leisure activities are beneficial for maintaining the health. In particular, the exercise such as sports is considered to be more beneficial for maintaining or improving the health, and hence is spread to not only athletes but also non-athletes.

However, exercise such as excessive sports may damage the muscle and may result in an adverse effect.
The damage of the muscle is caused by overload exercise. When a part of muscle is damaged, the cells in the damaged muscle are known to secrete cytokines or chemokines, and cause the infiltration of the neutrophil and macrophage. Then, the symptom accompanying an inflammation may be caused by such an amplifying action that reactive oxygen species generated by increase of the neutrophil, hypochlorous acid generated by the action of myeloperoxydase (hereinafter, maybe referred to as MPO) secreted from the neutrophil, and the like, damage the surrounding muscle. From the foregoing, the damage degree of the muscle due to overload exercise can be clarified by examining an MPO activity.

In addition, the damage degree of the muscle can be also clarified by examining the secretion amount or gene expression level with respect to interleukin-6 (hereinafter, may be referred to as IL-6) which is one of cytokines or neutrophil chemotactic factor-1 (hereinafter, may be referred to as CXCL-1) which is one of chemokines and involved in the migration of the neutrophil, or the like.

As a substance effective for the inflammation associated with the muscle damage, there are known non-steroidal anti-inflammatory compounds such as phenylbutazone, salicylic acid derivatives, indomethacin, and phenyl acetate.

However, the non-steroidal anti - inflammatory compounds have an action of aiding recovery and cure after the inflammation is caused, but does not have an effect of protecting or preventing the muscle damage.
Further, the non-steroidal anti-inflammatory compounds have side effects such as a damaging a muscular mucosa or a causing a gastric ulcer in a digestive system.
Therefore, in order to reduce the side effects, there is proposed a composition obtained by blending a phospholipid in the non-steroidal anti-inflammatory compound (refer to Patent Document 1, for example), but the composition has not reduced the side effect satisfactorily.

In order to obtain more effective action, there is demanded a substance which has a muscle damage-protecting function and has high safety with extremely little side effect. In the circumstances, vitamin E, for example, may inhibit the amplifying action of the muscle damage by scavenging the reactive oxygen species generated in overload exercise (refer to Non-patent Document 1, for example).
In addition, there is proposed that lycopene or the like inhibits a muscle protein degradation and has a muscle damage-protecting action (refer to Patent Document 2, for example).

Those substances may have higher safety, but is insufficient in the action of protecting and preventing the muscle damage.
Accordingly, there has been demanded development of a composition for protecting a muscle damage having high safety and additionally strong effect.

Patent Document 1: JP 55-89225 A
Patent Document 2: JP 2004-59518 A
Non-patent Document 1: Tohoku J. Exp. Med., Vol. 198, p. 47 to 53, 2002

### Disclosure of the Invention

### Problems to be solved by the Invention

An object of the present invention is to solve the above-mentioned problems of the conventional technologies and provide a composition for protecting a muscle damage having high safety and a stronger muscle damage-protecting effect.

### Means for solving the Problems

In view of the above problems, the inventors of the present invention focused on acetic acid bacteria which have been used as vinegar-producing bacteria since ancient time and confirmed to have high safety. Note that the acetic acid bacteria have been deeply related to the dietary life of the human since ancient time. For example, in addition to the example of the vinegar production, Caspian yogurt as a traditional food of Europe contains acetic acid bacteria and it is known that the acetic acid bacteria have been eaten historically.
Note that, the acetic acid bacteria are filtered and disposed after the completion of the fermentation in the vinegar manufacturing, and thus is available with inexpensive price.
Further, the inventors of the present invention also focused on vinegar fermented by the acetic acid bacteria. Vinegar has been a popular acid taste seasoning for a long time and there are given rice vinegar, grain vinegar, black vinegar, unpolished-rice vinegar, fruit vinegar such as apple cider vinegar and wine vinegar, and spirit vinegar.

The inventors have studied extensively. As a result, the inventors confirmed that the muscle damage-protecting action was found in the extract obtained from the acetic acid bacteria or vinegars with lipid-soluble organic solvents, thereby completing the present invention.
Further, the inventors confirmed that the muscle damage-protecting action was found in an alkali-stable lipid contained in the extract with lipid-soluble organic solvents of acetic acid bacteria, and in N-acylsphinganine, sphinganine, amino lipids, and terpenoid compounds, each of which constitutes the alkali-stable lipid, thereby completing the present invention.

That is, a first embodiment of the present invention provides a composition for protecting a muscle damage, comprising an extract with a lipid-soluble organic solvent of an acetic acid bacterium as an active ingredient.
In addition, a second embodiment of the present invention provides the composition for protecting a muscle damage according to the first embodiment, wherein the lipid-soluble organic solvent is ethyl acetate.
In addition, a third embodiment of the present invention provides the composition for protecting a muscle damage according to the first embodiment, wherein the extract with the lipid-soluble organic solvent is an alkali-stable lipid.
In addition, a fourth embodiment of the present invention provides the composition for protecting a muscle damage according to the third embodiment, wherein the alkali-stable lipid is N-acylsphinganine.
In addition, a fifth embodiment of the present invention provides the composition for protecting a muscle damage according to the third embodiment, wherein the alkali-stable lipid is sphinganine.
In addition, a sixth embodiment of the present invention provides the composition for protecting a muscle damage according to the third embodiment, wherein the alkali-stable lipid is an amino lipids.
In addition, a seventh embodiment of the present invention provides the composition for protecting a muscle damage according to the third embodiment, wherein the alkali-stable lipid is a terpenoid compounds.
Further, an eighth embodiment of the present invention provides a composition for protecting a muscle damage, comprising an extract with a lipid-soluble organic solvent of a vinegar as an active ingredient.
Still further, a ninth embodiment of the present invention provides the composition for protecting a muscle damage according to the eighth embodiment, wherein the lipid-soluble organic solvent is ethyl acetate.

### Effects of the Invention

The extract with a lipid-soluble organic solvent of an acetic acid bacterium or a vinegar of the present invention is orally ingested, whereby a strong muscle damage-protecting effect can be exhibited. Further, an alkali-stable lipid contained in the extract with a lipid-soluble organic solvent of an acetic acid bacterium, and in addition, N-acylsphinganine, sphinganine, amino lipids, and terpenoid compounds, each of which constitutes the alkali-stable lipid, are orally ingested, whereby a strong muscle damage-protecting effect can be exhibited.
Therefore, according to the present invention, a composition for protecting a muscle damage having high safety and a strong muscle damage-protecting effect can be provided. Oral ingestion of the composition for protecting a muscle damage according to the present invention as a food or the like is expected to have an effect of protecting a muscle damage due to sports or heavy exercise and a light muscle damage associated with physical activities in daily life.

### Brief Description of the Drawings

Fig.1 is a graph illustrating MPO activities in a myocardium, a soleus muscle, and a gastrocnemius muscle after light exercise of the present invention.
Fig. 2 is a graph illustrating an MPO activity in a myocardium under heavy exercise of the present invention.
Fig. 3 is a graph illustrating muscle damage-protecting effects of an extract with a lipid-soluble solvent of an acetic acid bacterium and an alkali-stable lipid of an acetic acid bacterium of the present invention.
Fig. 4 is a graph illustratingmuscle damage-protecting effects of N-acylsphinganine, sphinganine, amino lipids, and terpenoid compounds of the present invention.

### Best Mode for carrying out the Invention

Hereinafter, the present invention is described in detail.
The acetic acid bacterium used in the present invention is not particularly limited. For example, acetic acid bacteria belonging to *Gluconacetobacter, Gluconobacter, Acetobacter, Asaia,* or *Acidomonas* are exemplified.

More specifically, examples of the acetic acid bacterium belonging to *Gluconacetobacter* include *Gluconaeetobacter hansenii, Gluconacetobacter diazotrophicus, Gluconacetobacter intermedius, Gluconacetobacter sacchari, Gluconacetobacter xylinus, Gluconacetobacter europaeus,* and *Gluconacetobacter oboediens.*

Next, examples of the acetic acid bacterium belonging to Gluconobacter include *Gluconobacter frateurii* and *Gluconobacter cerinus.*

In addition, examples of the acetic acid bacterium belonging to *Acetobacter* include *Acetobacter tropicalis, Acetobacter indonesiensis, Acetobacter syzygii, Acetobacter cibinongensis, Acetobacter orientalis, Acetobacter pasteurianus, Acetobacter orleanensis, Acetobacter lovaniensis, Acetobacter aceti, and Acetobacter pomorum.*

Further, examples of the acetic acid bacterium belonging to *Asaia* include *Asaia bogorensis* and *Asaia siamensis.*

In addition, examples of the acetic acid bacterium belonging to *Acidomonas* include *Acidomonas methanolica.*

Further, as the acetic acid bacterium, in addition to the above-mentioned bacteria, an acetic acid bacterium used in a vinegar production or a fermented food such as yogurt, an acetic acid bacterium isolated from the nature, and a stock stain which is preserved in microbial culture collections and can be shared are appropriately used.

The acetic acid bacteria are used in the fermentation of acetic acid. In addition, the acetic acid bacteria have been eaten as natadecoco, Caspian yogurt, tea mushroom, and the like, and hence are recognized to have high safety. In particular, the acetic acid bacteria are filtered and disposed after the fermentation in the vinegar fermentation, and thus are available in large amount with inexpensive price.

In addition, the vinegar used in the present invention is not particularly limited. For example, a rice vinegar, a grain vinegar, a black vinegar, an unpolished-rice vinegar, fruit vinegar such as an apple cider vinegar and a wine vinegar, and a spirit vinegar are exemplified. Of those, the grain vinegar is preferred because the grain vinegar is produced using grains such as wheat, corn, rice, and the like, are generally used, and are relatively inexpensive.

In the present invention, as described in the first or eighth embodiment, a composition for protecting a muscle damage is constituted using an extract, as an active ingredient, extracted from the acetic acid bacterium or vinegar by using a lipid-soluble organic solvent. Further, as described in the third to seventh embodiments, a composition for protecting a muscle damage is constituted using an alkali-stable lipid prepared from an extract with a lipid-soluble organic solvent, or N-acylsphinganine, sphinganine, amino lipids, or terpenoid compounds in the alkali-stable lipid. The composition for protecting a muscle damage would contain the above components as an active ingredient, therefore an acetic acid bacterium treated with a cell disrupter or the like may be used.

Note that the treatment with a cell disrupter of an acetic acid bacterium may be conducted according to a known method, and is conducted by using an ultrasonic cell disrupter or a high pressure cell disrupter such as French press.

As the lipid-soluble organic solvent used in the present invention, a hydrophobic organic solvent such as ethyl acetate, phenol, and n-butyl alcohol, or a mixture solvent of the hydrophobic organic solvent and an hydrophilic organic solvent such as acetone, ethanol, methanol, propanol, isopropanol, or butanol, or an aqueous solution containing the solvent is used. Of those, as described in the second or ninth embodiment, ethyl acetate having a neutral polarity or a mixture solvent thereof is preferred.

Note that the extract with a lipid-soluble organic solvent from an acetic acid bacterium or a vinegar is prepared by direct extraction using the lipid-soluble organic solvent. In particular, in the case of preparation of the extract with a lipid-soluble organic solvent from an acetic acid bacterium, it is preferred to add, prior to the extracting operation with the lipid-soluble organic solvent, a step of removing precipitates due to a treatment of an acetic acid bacterium suspension, with a hydrophilic organic solvent such as acetone.

The alkali-stable lipid from the acetic acid bacterium can be prepared by subjecting lipids, which are extracted from the acetic acid bacteria using an organic solvent consisting of one kind or two or more kinds selected from ethanol, acetone, hexane, chloroform, methanol, ethyl acetate, and the like, to weak alkaline decomposition treatment to thereby remove phospholipids. In view of the fact that safety is required for use in food and a low polar solvent is suitable for efficient extraction, hexane and acetone are suitable for the extraction solvent.

The thus obtained alkali-stable lipid of the acetic acid bacteria contains, as components, N-acylsphinganine, sphinganine, aminolipids,terpenoid compounds,andfatty acids. Those compounds are general as membrane lipids derived from cells of acetic acid bacteria and are contained in the acetic acid bacteria belonging to *Acetobacter, Gluconobacter, Gluconacetobacter,* and the like, and the detail of the compounds is as follows.

That is, examples of the N-acylsphinganine include N-2'-hydroxypalmitoyl-sphinganine, O-1-glucuronyl-N-2'-hydroxypalmitoyl-sphinganine, N-cis-vaccenoyl-sphinganine, and 0-1-glucuronyl-N-cis-vaccenoyl-sphinganine.

Examples of the amino lipids include 3-(palmitoyl)-hydroxypalmitoyl-ornithyl-taurine as ornithyl tarurine lipid, 3-(palmitoyl)-hydroxypalmitoyl-ornithine as ornithine lipid, and 3-hydroxypalmitoyl-ornithine as lyso-ornithine lipid.

Examples of the terpenoid compounds (hopanoids) include tetrahydroxybacteriohopane(C35-pentacyclic terpene alcohol), hopane-22-ol, and hop-22(29)-ene.
Examples of the fatty acids include cis-vaccenic acid.

N-acylsphinganine refers to a kind of the sphingolipid contained in the alkali-stable lipid. The N-acylsphinganine is a compound generally named as ceramide in which a fatty acid binds to sphinganine as a sphingoid base, and is generally represented by the following chemical formula (1). Note that, in the formula (1), -CO-R represents an acyl group. In the formula (1), as the fatty acid substituting the acyl group, there are known hydroxy fatty acids or normal fatty acids, saturated fatty acids or unsaturated fatty acids, linear fatty acids or branched chain fatty acids, and fatty acids having 2 to 30 or less carbon atoms.

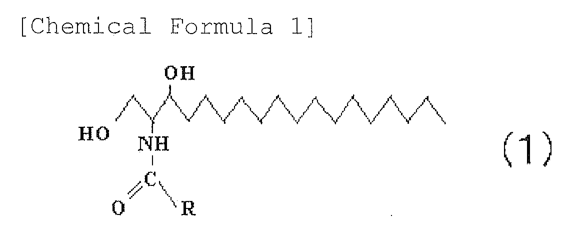

Note that, in the case of N-acylsphinganine derived from acetic acid bacteria, fatty acids each substituting an acyl group such as myristic acid, palmitic acid, stearic acid, palmitoleic acid, oleic acid, vaccenic acid, linoleic acid, 2-hydroxymyristic acid, and 2-hydroxy palmitic acid are confirmed. In particular, N-2'-hydroxypalmitoyl-sphinganine, N-palmitoyl-sphinganine, N-cis-vaccenoyl-sphinganine account for most of the content ratio of the N-acylsphinganine in the case of acetic acid bacteria (refer to "Doctor thesis of Graduate School of Agricultural Sciences, Iwate University", written by Hidetsugu Gotou, p. 11 to 41 (2001), for example).

The content ratios of the above compounds are different from one another depending on the kind of the acetic acid bacteria. The compounds are present as main constituents of membrane lipids only in Gram-negative bacteria to which the acetic acid bacteria belong and are not present as lipid components in other organisms such as Gram-positive bacteria and eukaryote. Alternatively, the compounds are present in extremely trace amount.

The extraction of a muscle damage-protecting substance with organic solvents from an acetic acid bacteria or vinegars for production of a composition for protecting a muscle damage may be conducted according to a known method. For example, after an acetic acid bacterium is appropriately disrupted using ultrasonic cell disrupter, the muscle damage-protecting substance is extracted with an organic solvent solution, and the resultant is then concentrated.
In the case of the vinegar, similarly, the muscle damage inhibitory substance is extracted with an organic solvent solution and the resultant is then concentrated. The concentrate as it is can be used directly as a composition for protecting a muscle damage. In addition, the muscle damage-protecting substance is purified by liquid chromatography, operating in a counter current distribution method, or the like, and the purified resultant is collected and can be used as the composition for protecting a muscle damage.

Further, for example, the muscle damage-protecting substance is used together with antioxidants such as vitamin E and coenzyme Q10, amino acids, soybean proteins, lactoproteins, and the like, to thereby be used as the composition for protecting a muscle damage of the present invention.

In addition, as the form of the composition for protecting a muscle damage of the present invention, a solution in which the composition for protecting a muscle damage is dissolved in water, alcohol, hydrous alcohol, or the like, a powder obtained by drying the solution, or a tablet obtained by molding the powder may be used. Further, the composition for protecting a muscle damage is used as supplement, beverage, food and drug for exercise, and the form thereof is not particularly limited.
As the form of the beverage or food, the composition for protecting a muscle damage is mixed in, specifically, a vegetable juice using a tomato, carrot, or the like as a raw material, a fruit juice using an apple, pineapple, grapefruit, orange, peach, or the like as a raw material, a mixture product of a vegetable juice and a fruit juice, an alcoholic beverage, a daily product such as milk and yogurt, a sport beverage, coffee, a tea product such as a black tea, green tea, and Oolong tea, a vinegar such as a black vinegar, a grain vinegar, a rice vinegar, an unpolished-rice vinegar, a black vinegar, a spirit vinegar, a fruit vinegar containing a fruit as a raw material such as an apple cider vinegar and a wine vinegar, and a vegetable vinegar containing a vegetable as a raw material, a confectionery such as a candy, gum, jelly, ice cream, and cookie, a staple food such as bread, rice, and noodles, or the like.

When the composition for protecting a muscle damage of the present invention is prepared in the above-mentioned form, another health functional component derived from a natural product is contained, whereby an additive effect or a synergistic effect is expected.

The dosage of the extract with the lipid-soluble organic solvent of acetic acid bacteria, the alkali-stable lipid of acetic acid bacteria, N-acylsphinganine, sphinganine, the amino lipids, the terpenoid compounds, or the extract with the lipid-soluble organic solvent of vinegars, as the composition for protecting a muscle damage of the present invention, is 0.001 mg to 100 g and preferably 0.1 mg to 10 g per adult per day. When the dosage is less than 0.001 mg per adult per day, the muscle damage cannot be protected sufficiently.

The composition for protecting a muscle damage of the present invention is produced by mixing uniformly with the extract with the lipid-soluble organic solvent of acetic acid bacteria, the alkali-stable lipid of acetic acid bacteria, N-acylsphinganine, sphinganine, the amino lipids, or the terpenoid compounds, or the extract with the lipid-soluble organic solvent of vinegars, as an active ingredient, various raw materials other than the active ingredient, and as required, a surfactant or the like to stabilize the composition.
Examples of the surfactant include sorbitan fatty acid ester, glycerin fatty acid ester, propylene glycol fatty acid ester, sucrose fatty acid ester, and lecithin.

The administering part of the composition for protecting a muscle damage of the present invention is not particularly limited as long as the part is a muscle. Examples thereof include skeletal muscles such as a soleus muscle and a gastrocnemius, a smooth muscle, a respiratory muscle, and a myocardium, and the skeletal muscles such as the soleus muscle and the gastrocnemius and the myocardium are particularly preferred.

The exercise intensity with which the muscle damage-protecting effect can be exhibited is not particularly limited. The exercise intensity may be 80% or less and particularly preferably 40 to 60% of a maximal oxygen uptake (VO₂ max).

The muscle damage-protecting effect of the present invention can be confirmed by the following method.
That is, an exercise system in which a rat is made to run with a treadmill was used. A treadmill is a running machine used by the human for the purpose of running or walking. In the present invention, a treadmill refers to a miniaturized machine dedicated for rats. The treadmill is capable of putting load on a muscle physically, which is different from a conventional exercise method by swimming compulsorily, to thereby examine strictly the effect on exercise.

In the present invention, MPO present in a granule of a neutrophil which is one of phagocytes was used as a marker of muscle damage upon exercise in the exercise system by running with the treadmill. The reason therefor is as follows. When the muscle is damaged due to exercise, it is known that the number of the neutrophils increases and the MPO in the neutrophils is secreted from them, and the MPO catalyzes hydrogen peroxide in the organism, to generate a highly reactive hypochlorous acid, to thereby cause additional damage to the peripheral tissues, and therefore, the degree of the damage due to the exercise can be clarified by measuring an MPO activity.

Further, the damage-protecting effect in the present invention in vitro was evaluated using a myotube cell. The myotube cell is differentiated from a myoblast and has a muscle fiber-like form. Note that the method can evaluate the effect on a muscle damage more directly than a method using an animal.

In the system using a myotube cell, as markers of the muscle damage, the amounts of cytokine IL-6 which are used as a general inflammatory marker and the amounts of chemokine CXCL-1 which is involved in the migration of the neutrophil were measured. Because it is known that not only a physical muscle damage but also an inflammatory reaction induced by the damage is involved largely in the exercise-induced muscle damage, and the damaged muscle cells may secrete cytokines or chemokines to promote the infiltration of the neutrophils or macrophages. From the foregoing, as markers of the muscle damage, the representations of IL-6 and CXCL-1 were measured.

### Examples

Next, the present invention is described in detail by way of examples, but the scope of the present invention is not limited thereby.

### Production Example 1 (Preparation of extract with lipid-soluble organic solvent of acetic acid bacterium in large amount)

As an acetic acid bacterial strain, *Gluconacetobacter xylinus* NBRC15237 strain was used. The acetic acid bacterial strain is preserved in National Institute of Technology and Evaluation, Department of Biotechnology, Biological Resource Center (2-5-8, Kazusakamatari, Kisarazu-shi, Chiba-ken) and the acetic acid bacterium can be shared.

First, an extract with a lipid-soluble organic solvent of the acetic acid bacterium was prepared by the following method. 1.8 kg of dry cells of the acetic acid bacterium were suspended in 35 L of water, and disrupted using a French press cell disrupter at 10, 000 psi. The suspension of the disrupted cells was mixed with acetone. Then, in order to remove the sediment produced by the mixing, filtration was conducted. The filtrate was mixed with acetone again and the sediment was removed. The operation was performed twice. The obtained filtrate was concentrated under reduced pressure, and the concentrate was added and mixed with ethyl acetate and water. Then, the mixture was separated to an organic layer and an aqueous layer, whereby the organic layer was collected. Ethyl acetate was added to the aqueous layer again, and the mixture was separated similarly, whereby the organic layer was collected. The organic layer was concentrated under reduced pressure and dissolved in ethanol. The resultant was concentrated under reduced pressure again, whereby 108 g of the extract with the lipid-soluble organic solvent of the acetic acid bacterium was obtained.

### Production Example 2 (Preparation of extract with lipid-soluble organic solvent of vinegar in large amount)

1, 200 L of ethyl acetate were mixed in 1, 000 L of a grain vinegar and the mixture was left to stand and separated into two layers.
Then, an organic layer was collected and concentrated under reduced pressure. When the volume of the organic layer was reduced to about 15 L, ethanol was loaded in an equivalent amount. The obtained mixture was stirred and further concentrated under reduced pressure, whereby 234 g of the extract with the lipid-soluble organic solvent of the vinegar were obtained.

### Production Example 3 (Preparation of alkali-stable lipid of acetic acid bacterium)

As an acetic acid bacterial strain, *Gluconacetobacter xylinus* NBRC15237 strain was used as in Production Example 1.

First, an alkali-stable lipid of the acetic acid bacterium was prepared by the following method. That is, 2, 000 g of dry cells of the acetic acid bacterial strain was subjected to extraction with a chloroform-methanol solvent (chloroform:methanol:water=1:2:0.8), followed by separation into two layers. The lower layer was collected as a crude lipid fraction. Next, the crude lipid fraction was subjected to a weak alkaline decomposition treatment with 0.4 N NaOH to remove phospholipids, and the resultant was extracted again with a solvent according to the composition of Folch (chloroform:methanol:water=8:4:3). The resultant was dried to solidness by evaporation, whereby the alkali-stable lipid was obtained.

The lipid components contained in the obtained alkali-stable lipid of the acetic acid bacterium were confirmed by thin layer chromatography using a silica gel plate. As a developing solvent in the thin layer chromatography, a solution having a ratio of chloroform to methanol of 96:4 was used for fatty acids, terpenoid compounds, and N-acylsphinganine, and a solution having a ratio of chloroform, methanol, to water of 65:16:2 was used for sphinganine and amino lipids.

As standard compounds of the thin layer chromatography, extracted products of an acetic acid bacterium confirmed to be terpenoid compounds, N-acylsphinganine, or amino lipids, or the like (for example, refer to "RESEARCH BULLETIN OF OBIHIRO UNIVERSITY", Vol. 23, p. 917 to 925 (1978), and "Doctor thesis of Graduate School of Agricultural Sciences, Iwate University", written by Hidetsugu Gotou, p. 11 to 41 (2001)) as well as a commercially available sphinganine (manufactured by SIGMA CORPORATION) were used. As a result of the thin layer chromatography, it was revealed that all compounds used as standard compounds were present in the alkali-stable lipid.

In addition, the contents of the main components in the alkali-stable lipid were confirmed by high performance liquid chromatography. That is, the alkali-stable lipid was reacted in the presence of anhydrous pyridine and benzoyl chloride at 70°C for 10 minutes, whereby benzoyl derivatives of terpenoid compounds and N-acylsphinganine were purified from the resultant. The benzoyl derivatives were subjected to high performance liquid chromatography and were detected by an ultraviolet absorption at a wavelength of 230 nm. The content of each compound present in the alkali-stable lipid was calculated from a standard curve obtained by using commercially available compounds or the standard compounds purified from the acetic acid bacterium of which the chemical structure had been confirmed. As a mobile phase of the high performance liquid chromatography, a solvent having a ratio of hexane to isopropanol of 100:1 was used and a flow rate was set to 1 ml/minute.

Table 1 below shows the thus analyzed main components in the alkali-stable lipid of acetic acid bacterium.
Note that the presence of the amino lipids was confirmed (the content ratio is unconfirmed), but the presence of phospholipids such as phosphatidylcholine, phosphatidylserine, and phosphatidyl glycerol was not confirmed.

**[Table 1]**

| Main component of alkali- stable lipid of acetic acid bacterium | Content ratio (weight/weight%) |
|---|---|
| Terpenoid compounds | 11.511 |
| N-acylsphinganine | 5.446 |
| Sphinganine | 3.173 |

### Production Example 4 (Purification of terpenoid compounds, N-acylsphinganine, amino lipids, and sphinganine)

10 g of the alkali-stable lipid was loaded onto a silica gel column equilibrated with chloroform. The column was washed with a solvent prepared by mixing chloroform and acetic acid at a volume ratio of 99:1, and the elution was performed with a solvent having a ratio of chloroform to methanol of 97:3. The obtained fraction was dried to solidness, whereby a fraction 1 was obtained.

Subsequently, the fraction obtained by elution with solvents each having a ratio of chloroform to methanol of 96:4 and 95:5 was dried to solidness, whereby a fraction 2 was obtained. Further, the fraction obtained by eluting the lipids with solvents each having a ratio of chloroform to methanol of 92:8 and 9:1 was dried to solidness, whereby a fraction 3 was obtained. Further, the fraction obtained by elution with a solvent having a ratio of chloroform to methanol of 2:1 was dried to solidness, whereby a fraction 4 was obtained.

The constituent lipids of the obtained fraction 1 were confirmed by thin layer chromatography using a silica gel plate. As the developing solvent in the thin layer chromatography, a solvent having a ratio of chloroform to methanol of 96:4 was used. As a result, the constituent lipids of the fraction 1 showed a single spot which had the same mobility as that of the purified product from the acetic acid bacterium identified to be N-acylsphinganine used in Example 1. Further, the fraction 1 was decomposed with hydrous methanol-hydrochloride according to a known method and then separated into a sphingoid base and a fatty acid by thin layer chromatography, followed by purification.

To each sphingoid base and fatty acid, according to a known method, trimethyl silylation was performed and the chemical structure thereof was analyzed by gas chromatography and mass spectrometry. As a result, the fraction 1 was confirmed to contain N-2'-hydroxypalmitoyl-sphinganine, N-palmitoyl-sphinganine, and N-cis-vaccenoyl-sphinganine as N-acylsphinganine.
From the above results, the single spot detected by the thin layer chromatography of the fraction 1 was confirmed to be N-acylsphinganine.

Further, the constituent lipids in the fraction 2, 3 and 4 were confirmed by the thin layer chromatography. As the developing solvent in the thin layer chromatography, a solvent having a ratio of chloroform to methanol of 96:4 was used for the fraction 2, and solvents having a ratio of chloroform, methanol, to water of 65:16:2 were used for the fractions 3 and 4. As standards, the terpenoid compounds used in Production Example 3, and the purified amino lipids from the acetic acid bacterium of which the chemical structures had been identified, and a commercially available sphinganine (manufactured by SIGMA CORPORATION) were used.

As a result of the thin layer chromatography, the fraction 2 showed a spot corresponding to that of the terpenoid compounds as a standard, the fraction 3 showedmain spot corresponding to that of sphinganine as a standard, and the fraction 4 showed a main spot corresponding to that of the amino lipids as a standard.
From the above results, it was confirmed that the spot detected in the thin layer chromatography of the fraction 2 contained the terpenoid compounds, the spot detected in the thin layer chromatography of the fraction 3 contained sphinganine, and the spot detected in the thin layer chromatography of the fraction 4 contained the amino lipids.

### Example 1 (Muscle damage-protecting effect after light exercise)

The Crl:CD (SD) rats were exercised with a treadmill running (belt gradient: 6°, rate: 10 m/min) for 10 minutes per day for 1 week. After the exercise, samples were administered to the rats by oral ingestion for 1 week.
As a basic feed, a solid feed of CRF-1 (manufactured by Oriental Yeast Co. , Ltd.) was used. The rats were divided into the following three groups (n=8): a group in which only a basic feed was fed (non-administration group); a group in which the basic feed containing the extract with the lipid-soluble organic solvent of the acetic acid bacterium produced in Production Example 1 at the ratio of 500 mg/kg of rat body weight were compulsorily administered (acetic acid bacterium extract group); and a group in which the basic feed containing the extract with the lipid-soluble organic solvent of the vinegar produced in Production Example 2 at the ratio of 500 mg/kg of rat body weight were compulsorily administered (vinegar extract group). In addition, a disposable polypropylene syringe equipped with a disposable oral tube for a rat was used for the compulsory administration.

On day 7 after the administration, the rats were killed and the tissue was harvested. After then, MPO activities of the myocardium, soleus muscle, and gastrocnemius were measured. The MPO activities were measured using a Myeloperoxidase Assay kit (manufactured by CytoStore Inc.).
That is, 250 µl of hexadecyltri-methylammoniumbromide solution were added to about 0.5 g of each tissue. The tissue was disrupted with a mortar and the resultant was centrifuged at 10, 000 rpm, and a supernatant was collected. The collected supernatant was adjusted to have a tissue protein concentration of 20 mg/ml to obtain a tissue lysate for an MPO activity measurement. Note that the tissue protein concentration was measured using Bio Rad DC-protein Assay Kit (manufactured by Bio-Rad Laboratories).
Next, in order to prepare a reaction solution, 0.5 ml of 1% hydrogen peroxide was added to 100 ml of phosphate buffer containing o-dianisidine dihydrochloride. 200 µl of the reaction solution were mixed to 20 µl of the tissue lysate for an MPO activity measurement of each tissue. Then, each absorbance at 450 nm immediately after and 1 minute after the mixing was measured using an absorption spectrometer.
Note that increase in an absorbance (450 nm) per minute in the case where 1 M hydrogen peroxide was reacted per g of the tissue protein was defined as 1 U of the MPO activity.

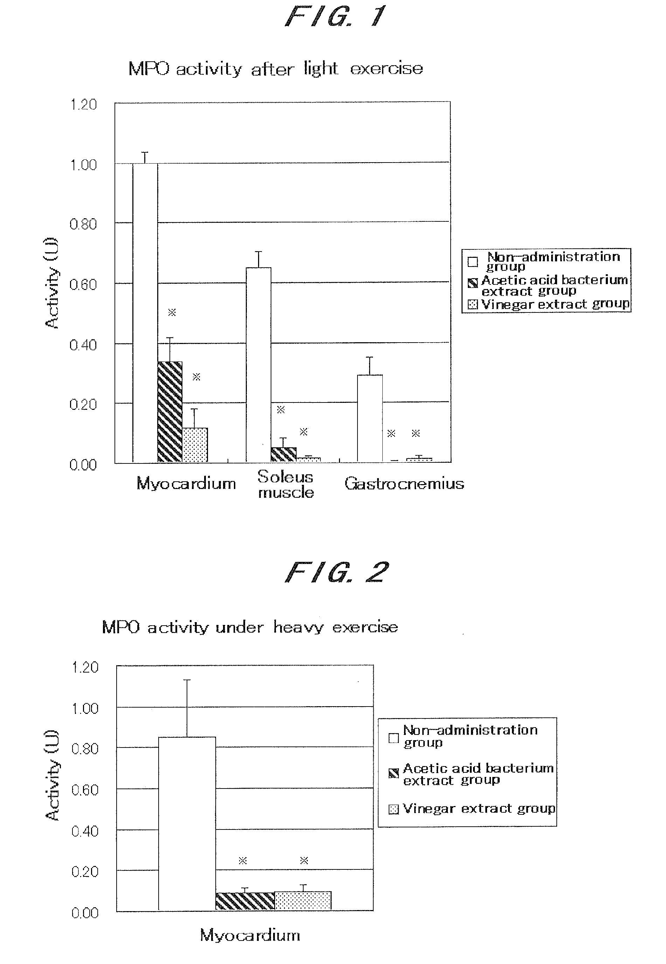

Fig. 1 shows the above results. Note that a t-test was performed with 5% significance level for the acetic acid bacterium extract group or vinegar extract group in comparison with the non-administration group of each tissue. The each group where significant difference was confirmed was represented by 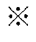 marks.

From the results in Fig. 1, in the acetic acid bacterium extract group and vinegar extract group, the MPO activity of the myocardium, soleus muscle, and gastrocnemius significantly decreased compared to that in the non-administration group. It was confirmed that both the extract with the lipid-soluble organic solvent of the acetic acid bacterium and the extract with the lipid-soluble organic solvent of the vinegar had the muscle damage-protecting action.

### Example 2 (Muscle damage-protecting effect under heavy exercise)

In order to examine the difference of the effect according to the intensity of exercise, the rats were accustomed to a treadmill running (belt gradient: 6°, rate: 10 m/min) for 10 minutes per day for 1 week. And then, no exercise was performed for the following 6 days, and on day 7, the rats were exercised with a treadmill running (belt gradient: 10°, rate: 20 m/min) for 10 minutes. In the period, the rats were administered each feed by compulsory oral ingestion
As a basic feed, a solid feed of CPF-1 (manufactured by Oriental Yeast Co. , Ltd.) was used. The rats were divided into the following three groups (n=8): a group in which only a basic feed was fed (non-administration group); a group in which the basic feed containing the extract with the lipid-soluble organic solvent of the acetic acid bacterium produced in Production Example 1 at the ratio of 500 mg/kg of rat body weight were compulsorily administered (acetic acid bacterium extract group); and a group in which the basic feed containing the extract with the lipid-soluble organic solvent of the vinegar produced in Production Example 2 at the ratio of 500 mg/kg of rat body weight were compulsorily administered (vinegar extract group). On day 7 after the administration, the rats were killed and the tissue was harvested 2 hours after the completion of the exercise. After then an MPO activity of the myocardium was measured. The measurement method was the same way as in Example 1.
Fig. 2 illustrates the above results. Note that a t-test was performed with 5% significance level for the acetic acid bacterium extract group and vinegar extract group in comparison with the non-administration group. The each group where significant difference was confirmed was represented by 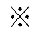 marks.

From the results in Fig. 2, the MPO activity in both of the acetic acid bacterium extract group and vinegar extract group significantly decreased compared to that in the non-administration group. In addition, it was confirmed that both the extract with the lipid-soluble organic solvent of the acetic acid bacterium and the extract with the lipid-soluble organic solvent of the vinegar protected the muscle damage. In addition, it was confirmed that the effect was exhibited even with increase in the intensity of exercise.

### Example 3 (muscle damage-protecting effect using myotube cell)

In this evaluation system, the C2C12 myoblast cell line derived from a mouse was used. The cell line is preserved in RIKEN BIORESOURCE CENTER, Japan, and the cell line can be shared. The cell is differentiated from a mononuclear myoblast cell into a maltinuclear myotube cell by differentiation induction, to thereby have a muscle fiber-like form. The cell was seeded in a collagen I-coated 35-mm dish and cultured in DMEM (11965-092, manufactured by GIBCO)+10% FBS (hereinafter, may be referred to as a growth medium) at 37°C under 5% CO₂ until the culture had 90% confluence. Then, the resultant culture was cultured in DMEM (11885-084, manufactured by GIBCO)+5% HS medium (hereinafter, may be referred to as a differentiation medium) at 37°C under 5% CO₂ for 72 hours so that the cell was differentiated into a myotube cell. Note that the medium was changed once 48 hours after the starting of the culture.

The extract with the lipid-soluble organic solvent of the acetic acid bacterium produced in Production Example 1 and the alkali-stable lipid of the acetic acid bacterium produced in Production Example 3, the terpenoid compounds, N-acylsphinganine, amino lipids, and sphinganine produced in Production Example 4 were each dissolved in DMSO, and each solution was added to the differentiation medium so as to have a final concentration of 10 µg/ml. Taking the influence of DMSO on the cell into consideration, the final concentration of DMSO after the addition to the medium was adjusted to 5 µl/ml. After the addition, the cell was cultured at 37°C under 5% CO₂ for 24 hours.

The myotube cell which was added with each lipid and cultured for 24 hours was washed with PBS three times and cultured in 1mM H₂O₂+differentiation medium at 37°C under 5% CO₂ for 2 hours. Then, the medium was disposed and the myotube cell was harvested. Note that, in the case where the harvested cell was not used immediately, the cell was subjected to flash freezing by using liquid nitrogen, and then stored in a freezer at -80°C until the use.

In order to analyze the gene expression of IL-6 and CXCL-1 of the harvested cell, the total RNA was isolated from the cell, a cDNA was synthesized by a reverse transcription, and the gene expression was analyzed by a quantitative real time PCR. That is, the total RNA was extracted roughly using Trizol solution containing phenol and guanidine isothiocyanate (manufactured by Invitrogen Corporation) from the harvested cell. After that, the total RNA was purified using SV total RNA isolation system (manufactured by Promega Corporation) according to an attached protocol and the purified total RNA was used for the reverse transcription. The concentration of the purified total RNA was determined based on the measurement results of the absorbance (260 nm, 280 nm).

The reaction solution in the reverse transcription was prepared based on the attached protocol by using Taqman Reverse Transcription Reagent. Note that the amount of the total RNA was 200 ng per sample. The condition of PCR for the reverse transcription was as follows: 25°C (10minutes) ; 48°C (30minutes) ; 95°C (5minutes) ; and 4°C. Thus, a cDNA was obtained.

A quantitative real time PCR was performed by using the obtained cDNA. In the quantitative real time PCR, qPCR super mix-UDG with ROX was used as a reaction reagent, as primers for IL-6 and CXCL-1, Mm00446190 (manufactured by AB Corporation) and Mm00433859 (manufactured by AB Corporation) were used, respectively, and as a primer for glycerol triphosphate dehydrogenase (hereinafter, may be referred to as GAPDH) which was used as an internal standard, Mm99999915 (manufactured by AB Corporation) was used. Then, a PCR reaction solution was prepared according to the protocol. The quantitative real time PCR was conducted using ABI prism 7000 with the following reaction condition: 50°C for 2 minutes; 95°C for 10 minutes; 40 cycles of 95°C for 15 seconds and 60°C for 1 minute; and 4°C. Each measurement value of IL-6 and CXCL-1 was represented by a relative value when the measurement value of GAPDH was 100.

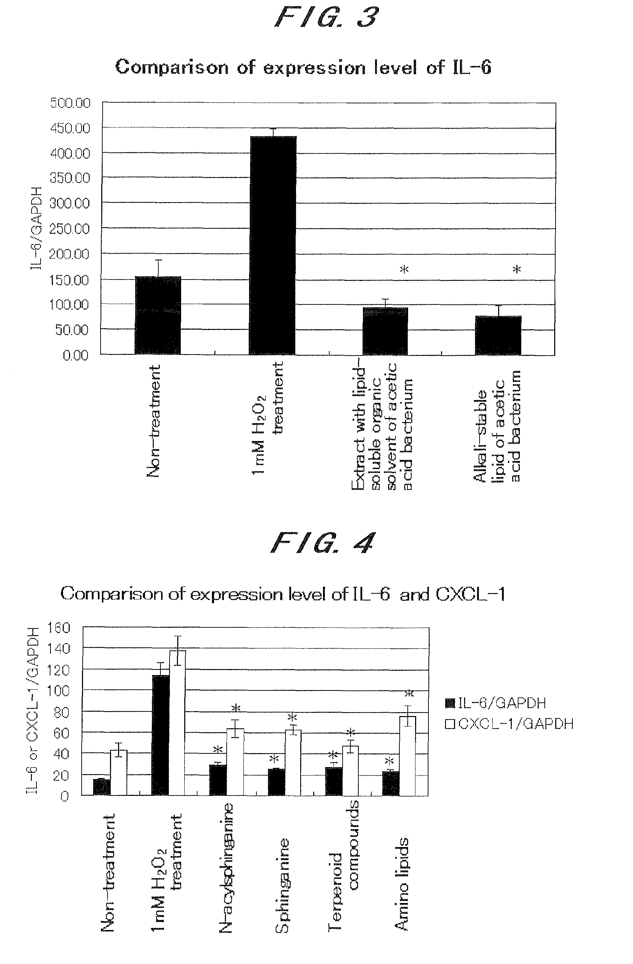

Figs. 3 and 4 illustrate the results. Note that a t-test was performed with 5% significance level, compared to the treatment group with hydrogen peroxide, for each of the treatment groups with the extract with the lipid-soluble organic solvent of the acetic acidbacterium, the alkali-stable lipid of the acetic acid bacterium, N-acylsphinganine, sphinganine, amino lipids, and terpenoid compounds. The test groups where significant difference was confirmed was represented by * marks.

From the results in Fig. 3, the extract with the lipid-soluble organic solvent of the acetic acid bacterium and the alkali-stable lipid of the acetic acid bacterium were confirmed to have an effect of suppressing the gene expression of IL-6.
From the results in Fig. 4, theN-acylsphinganine, sphinganine, amino lipids, and terpenoid compounds were confirmed to have an effect of inhibiting each of the gene expression of IL-6 and CXCL-1.
From the above results, each of the extract with the lipid-soluble organic solvent of the acetic acid bacterium, the alkali-stable lipid of the acetic acid bacterium, sphingolipid, sphinganine, amino lipids, and terpenoid compounds of the present invention was confirmed to have the muscle damage-protecting effect.

### Example 4 (Production of tablet)

### (1) Extract with lipid-soluble organic solvent of acetic acid bacterium

Cells in 10 kL of acetic acid-fermented broth were collected with a high speed centrifuge (8,000 rpm, 20 minutes), whereby 10 kg of wet cells were obtained. 10 kg of the obtained wet cells were washed with distilled water, and the resultant was then freeze-dried to solidness under reduced pressure with a large freeze-drying apparatus, whereby 1.8 kg of dry cells were obtained.

1 kg of the obtained dry cells was loaded together with 10 L of acetone into a Soxhlet extraction apparatus, followed by heat reflux for 20 hours. The obtained extracted solution was dried to solidness under reduced pressure. 10 L of ethyl acetate and 10 L of distilled water were mixed thereto and the mixture was separated into two layers. The organic solvent layer recovered was evaporated with a rotary evaporator and, when the volume was reduced to about 1 L, ethanol was loaded in an equal amount, followed by mixing. The mixture was dried to solidness by evaporation with a rotary evaporator. Thus, about 50 g of a pale yellowish brown extract with the lipid-soluble organic solvent of an acetic acid bacterium were obtained. 1 g (0.7 wt%) of the obtained extract with lipid-soluble organic solvent of the acetic acid bacterium was added with 35 g (26.9 wt%) of crystalline cellulose, 67 g (51.5 wt%) of dry corn starch, 22 g (16.9 wt%) of lactose, 2 g (1.5 wt%) of calcium stearate, and 3 g (2.3 wt%) of polyvinyl pyrrolidone as a binder agent to produce a mixed powder. The obtained powder was filled in a gelatin hard capsule.

### (2) Extract with lipid-soluble organic solvent of vinegar

5 L of a vinegar and 6 L of ethyl acetate were mixed and an organic solvent layer was evaporated with a rotary evaporator. When the volume was reduced to about 1 L, ethanol was mixed in an equal amount, and the mixture was further dried to solidness by evaporation with a rotary evaporator. Thus, about 1 g of a dark brown extract with the lipid-soluble organic solvent of the vinegar was obtained.
1 g (0.7 wt%) of the obtained extract with the lipid-soluble organic solvent of the vinegar was added with 35 g (26.9 wt%) of crystalline cellulose, 67 g (51.5 wt%) of dry corn starch, 22 g (16.9 wt%) of lactose, 2 g (1.5 wt%) of calcium stearate, and 3 g (2.3 wt%) of polyvinyl pyrrolidone as a binder agent to produce a mixed powder. The obtained powder was filled in a gelatin hard capsule.

Thus, it is expected that the tablets prepared in the sections (1) and (2) each can be used effectively for oral ingestion as a composition for protecting a muscle damage.

### Example 5 (Production of jelly)

### (1) Acetic acid bacterium-disrupted powder

Cells in 10 kL of acetic acid-fermented broth were collected with a high speed centrifuge (8,000 rpm, 20 minutes), whereby 10 kg of wet cells were obtained. 10 kg of the obtained wet cells were suspended in an equivalent amount of distilled water. 20 kg of the suspension of the acetic acid bacterium was subjected to cell disrupting treatment by being passed through a high pressure homogenizer (20,000 psi) three times. After that, the resultant was freeze-dried to solidness under reduced pressure with a large freeze-drying apparatus. Thus, 1.5 kg of a dry cell powder was obtained.

8 g (0.8 wt%) of the obtained dry cell powder, 250 g (25 wt%) of sugar, 1.6 g (0.16 wt%) of carageenan, 0.8 g (0.08 wt%) of locust bean gum, and 0.8 g (0.08 wt%) of xanthan gum were uniformly mixed, whereby a powder mixture was obtained. 300 g of water were measured in a pot, 30 g (3.0 wt%) of brown sugar were dissolved therein, and 150 g (15 wt%) of a hydrogenated syrup were mixed therein, and the powder mixture previously obtained was mixed and stirred until no lumps remained, followed by further stirring. Those compounds were stirred uniformly and 258.8 g of the additional water were added thereto, followed by heating. Those compounds were dissolved by heating at the temperature of 85°C for 10 minutes. After that, the resultant was cooled with running water, whereby jelly food containing the cells of the acetic acid bacterium was obtained.

### (2) Extract with lipid-soluble organic solvent of vinegar

50 L of a vinegar and 60 L of ethyl acetate were mixed and an organic solvent layer was evaporated with a rotary evaporator. When the volume was reduced to about 10 L, ethanol was mixed in an equal amount, and the mixture was further dried to solidness by evaporation with a rotary evaporator. Thus, about 10 g of a dark brown extract with the lipid-soluble organic solvent of the vinegar were obtained.

8 g (0.8 wt%) of the obtained extract with the lipid-soluble organic solvent of the vinegar, 250 g (25 wt%) of sugar, 1.6 g (0.16 wt%) of carageenan, 0.8 g (0.08 wt%) of locust bean gum, and 0.8 g (0.08 wt%) of xanthan gum were uniformly mixed, whereby a powder mixture was obtained. 300 g of water were measured in a pot, 30 g (3.0 wt%) of brown sugar were dissolved therein, and 150 g (15 wt%) of a hydrogenated syrup were mixed therein, and the powder mixture previously obtained was mixed and stirred until no lumps remained, followed by further stirring. Those compounds were stirred uniformly and 258.8 g of the additional water were added thereto, followed by heating. Those compounds were dissolved by heating at the temperature of 85°C for 10 minutes. After that, the resultant was cooled with running water, whereby a jelly food containing the extract with the lipid-soluble organic solvent of the vinegar was obtained.

Thus, it is expected that the jelly food prepared in the sections (1) and (2) each can be used effectively for oral ingestion as a composition for protecting a muscle damage.

### Example 6 (Production of beverage)

### (1) Vinegar containing acetic acid bacterium

Cells in 10 kL of acetic acid-fermented broth were collected with a high speed centrifuge (8,000 rpm, 20 minutes), whereby 10 kg of wet cells were obtained. 10 kg of the obtained wet cells were suspended in 100 L of vinegar. The suspension of the acetic acid bacterium was subjected to cell disrupting treatment by being passed through a high-pressure homogenizer (20, 000 psi) three times. The obtained suspension was dispensed in a 500-ml volume bottle, and the bottle was sterilized by heating to 75°C. Thus, a vinegar containing the cells of the acetic acid bacterium was obtained.

### (2) Vinegar containing extract with lipid-soluble organic solvent of vinegar at high concentration

500 L of a vinegar and 600 L of ethyl acetate were mixed and an organic solvent layer was evaporated with a rotary evaporator. When the volume was reduced to about 50 L, ethanol was mixed in an equal amount, and the mixture was further dried to solidness by evaporation with a rotary evaporator. Thus, about 100 g of a dark brown extract with the lipid-soluble organic solvent of the vinegar (vinegar lipid-soluble solvent fraction) were obtained. 100 g of the obtained vinegar lipid-soluble solvent fraction were dispersed in 1 L of a vinegar. The obtained solution was dispensed in a 500-ml volume bottle, and the bottle was sterilized by heating to 75°C. Thus, a vinegar containing the extract with the lipid-soluble organic solvent of the vinegar at high concentration was obtained.

It is expected that the beverages prepared in the sections (1) and (2) each can be used effectively for oral ingestion as a composition for protecting a muscle damage.

### Industrial Applicability

The present invention is capable of providing an oral product having high safety to a human body and the muscle damage-protecting effect at an inexpensive price. Accordingly, by oral ingestion of the composition for protecting a muscle damage of the present invention as a food or the like, there is expected an effect of protecting a muscle damage due to sports or heavy exercise and a light muscle damage associated with physical activities in daily life.

## Claims

1. A composition for protecting a muscle damage, comprising an extract with a lipid-soluble organic solvent of an acetic acid bacterium as an active ingredient.

2. A composition for protecting a muscle damage according to claim 1, wherein the lipid-soluble organic solvent is ethyl acetate.

3. A composition for protecting a muscle damage according to claim 1, wherein the extract with the lipid-soluble organic solvent is an alkali-stable lipid.

4. A composition for protecting a muscle damage according to claim 3, wherein the alkali-stable lipid is N-acylsphinganine.

5. A composition for protecting a muscle damage according to claim 3, wherein the alkali-stable lipid is sphinganine.

6. A composition for protecting a muscle damage according to claim 3, wherein the alkali-stable lipid is amino lipids.

7. A composition for protecting a muscle damage according to claim 3, wherein the alkali-stable lipid is terpenoid compounds.

8. A composition for protecting a muscle damage, comprising an extract with a lipid-soluble organic solvent of a vinegar as an active ingredient.

9. A composition for protecting a muscle damage according to claim 8, wherein the lipid-soluble organic solvent is ethyl acetate.
